(19) Europäisches Patentamt
European Patent Office
Office européen des brevets

(11) EP 0 570 764 B1

(12) EUROPÄISCHE PATENTSCHRIFT

(45) Veröffentlichungstag und Bekanntmachung des Hinweises auf die Patenterteilung:
**18.07.2001 Patentblatt 2001/29**

(21) Anmeldenummer: **93107272.2**

(22) Anmeldetag: **05.05.1993**

(51) Int Cl.[7]: **C07D 487/04**, C07D 231/04, C07D 237/04, C07D 243/02, C07D 231/06
// (C07D487/04, 237:00, 237:00)

# (54) Asymmetrische Hydrierung

Asymmetric hydrogenation

Hydrogénation asymétrique

(84) Benannte Vertragsstaaten:
**AT BE CH DE DK ES FR GB IT LI NL**

(30) Priorität: **18.05.1992 CH 158292**
**11.03.1993 CH 72993**

(43) Veröffentlichungstag der Anmeldung:
**24.11.1993 Patentblatt 1993/47**

(73) Patentinhaber: **F.HOFFMANN-LA ROCHE & CO. AKTIENGESELLSCHAFT**
**CH-4002 Basel (CH)**

(72) Erfinder:
- **Broger, Emil Albin**
  **CH-4312 Magden (CH)**
- **Crameri, Yvo**
  **CH-4104 Oberwil (CH)**
- **Imfeld, Marquard**
  **CH-4102 Binningen (CH)**
- **Montavon, François**
  **CH-2800 Delémont (CH)**
- **Widmer, Erich**
  **CH-4142 Münchenstein (CH)**

(74) Vertreter: **Witte, Hubert, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 408 340** **WO-A-93/15091**

- **HETEROCYCLES Bd. 7, Nr. 1 , 1977 Seiten 119 - 122 C.H. HASSALL ET AL. 'The Synthesis of (3S, 5S)-5-Hydroxyhexafydropyridazine-3-carboxy lic Acid'**
- **JOURNAL OF THE CHEMICAL SOCIETY, PERKIN TRANSACTIONS 1 1984 , LETCHWORTH GB Seiten 155 - 166 C.H. HASSALL ET AL. 'The Design and Synthesis of New Triazolo, Pyrazolo- and and Pyridazo-pyridazine Derivatives as Inhibitors of Angiotensin Concerting Enzyme'**
- **JOURNAL OF THE CHEMICAL SOCIETY, CHEMICAL COMMUNICATIONS Nr. 13 , Juli 1985 , LETCHWORTH GB Seiten 922 - 924 T. IKARYA ET AL. 'Synthesis of Novel Chiral Ruthenium Complexes of 2,2'-Bis(dip henylphosphino)-1,1'-binaphthyl and their Use as Asymmetric Catalysts'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 570 764 B1

Printed by Jouve, 75001 PARIS (FR)

## Beschreibung

Die vorliegende Erfindung betrifft ein neues katalysiertes Verfahren zur Herstellung von Verbindungen der allgemeinen Formel

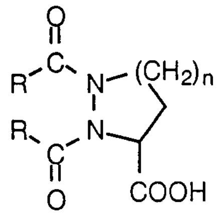

I

worin
jedes

R unabhängig Alkyl, Arylmethyl, Aryl, Alkoxy, Arylmethoxy oder Aryloxy ist oder beide R zusammen Methylen, Aethylen oder 1,2-Phenylen bedeuten, wobei der Term Aryl allein oder in Kombination Phenyl oder substituiertes Phenyl ist, wobei substituiertes Phenyl ein Phenyl mit einem oder mehreren, gleichen oder verschiedenen Substituenten ausgewählt aus $C_1$-$C_6$-Alkyl, $C_1$-$C_6$-Alkoxy oder Halogen ist; und
n 1, 2 oder 3 bedeuten,

in der (S)- oder (R)-Form, die wertvolle, zum Teil bekannte Zwischenprodukte sind.

Das erfindungsgemässe Verfahren ist dadurch gekennzeichnet, dass man das entsprechende 3-Carboxy-3-pyrazolin-, 3-Carboxy-1,2,5,6-tetrahydropyridazin- bzw. 3-Carboxy-1,2-diaza-3-cyclohepten-Derivat der allgemeinen Formel

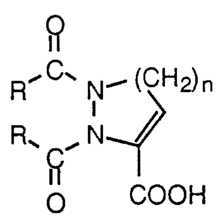

I

worin die beiden R und n die oben angegebenen Bedeutungen besitzen,
oder ein Salz davon in Gegenwart eines optisch aktiven Ruthenium-Diphosphin-Komplexes asymmetrisch hydriert, wobei die Umsetzung von 3,4,6,11-Tetrahydro-6,11-dioxo-pyridazo[1,2-a]phthalazin-1-carbonsäure zu (S)-1,2,3,4,6,11-Hexahydro-6,11-dioxo-pyridazo[1,2b]phthalazin-1-carbonsäure mittels eines Katalysators erhältlich aus Di($\eta^2$-acetato-($\eta^4$-cycloocta-1,5-dien)-ruthenium(II) und [(S)-6,6'-Dimethoxybiphenyl-2,2'-diyl]bis[diisopropyl-phosphin] ausgeschlossen ist.

Das Dokument, Heterocycles 7 (1977), 119-122, beschreibt die asymmetrische Hydrierung eines Tetrahydro-9,10-dioxo-pyridazino[1,2-b]-phthalazin-1-carbonsäureesters mittels eines Rhodiumkatalysators zur Hexahydro-Verbindung. Das Dokument, J.Chem.Soc.Perkin Trans. 1(1984), 155-156, beschreibt die Hydrierung von 1,4,6,9-Tetrahydro-6,9-dioxopyridazo[1,2-a]pyridazin-1-carbonsäuremethylester mittels eines Platinoxid-katalysators zur entsprechenden Octahydro-Verbindung, wobei kein Asymmetriezentrum geschaffen wird. Die Dokumente, EP-A-0 408 340, und, J.Chem.Soc.Chem.Comm. 13 (1985)922-924, beschreiben die Verwendung von Komplexen aus Ruthenium und optisch aktiven Phosphinen als Katalysatoren für asymmetrische Synthesen, z.B. von optisch aktiven Hydrozimtsäurestern. Das Dokument, WO-A-93/15091, beschreibt die Hydrierung von 3,4,6,11-Tetrahydro-6,11-dioxo-pyridazo [1,2-a]phthalazin-1-carbonsäure mittels eines Katalysators erhältlich aus Di($\eta^2$-acetato-($\eta^4$-cycloocta-1,5-dien)ruthenium(II) und [(S)-6,6'-Dimethoxybiphenyl-2,2'-diyl]-bis[diisopropylphosphin] zur entsprechenden (S)1,2,3,4,6,11-Hexahydro-Verbindung.

Bei den $C_{1-6}$-Alkylgruppen können die grösseren ($C_{3-6}$-)Alkylgruppen geradkettig oder verzweigt sein. Beispiele solcher Alkylgruppen sind Methyl, Aethyl, Propyl, Isopropyl, n-Butyl, Isobutyl, tert.Butyl, n-Pentyl, tert.Pentyl, Neopentyl, n-Hexyl und dergleichen. Der Ausdruck "Alkoxy" bedeutet derartige Gruppen, in denen der Alkylrest die

vorhergehende Bedeutung hat. Als Salze der Carboxyverbindungen der Formel II kommen insbesondere in Betracht Alkalimetall-, Erdalkalimetall- sowie Ammoniumsalze, wie beispielsweise das Natrium-, Kalium-, Calcium-, Magnesium- und Triäthylammoniumsalz.

Im Rahmen der vorliegenden Erfindung bedeutet ferner das Zeichen "▬", dass sich der entsprechende Substituent oberhalb der Molekülebene befindet.

Als Ruthenium-Katalysatoren (optisch aktive Ruthenium-Diphosphin-Komplexe) für das erfindungsgemässe Verfahren kommen insbesondere in Frage diejenigen Komplexe der allgemeinen Formeln

$$Ru(X^1)_2L \qquad III$$

und

$$[Ru(X^2)_{2-m}X^3L](X^4)_m \qquad IV$$

worin

- $X^1$ Halogenid, ein Anion $A\text{-}COO^{\ominus}$ oder ein Anion $A\text{-}SO_2O^{\ominus}$,
- $X^2$ Halogenid,
- $X^3$ Benzol, Hexamethylbenzol oder p-Cymol,
- $X^4$ Halogenid, $BF_4^{\ominus}$, $ClO_4^{\ominus}$ oder $B(Phenyl)_4^{\ominus}$,
- m die Zahl 1 oder 2,
- A $C_1$-$C_4$-Alkyl, halogeniertes $C_1$-$C_4$-Alkyl oder Aryl ist, wobei der Term Aryl ein Phenyl, Biphenyl, Naphthyl, substituiertes Phenyl, substituiertes Biphenyl oder substituiertes Naphthyl ist, wobei die substituierenden Gruppen ein oder mehrere, gleiche oder verschiedene Substituenten ausgewählt aus $C_1$-$C_4$-Alkyl oder Halogen sind und
- L einen optisch aktiven atropisomeren Diphosphinliganden bedeutet, insbesondere einen Liganden der allgemeinen Formel

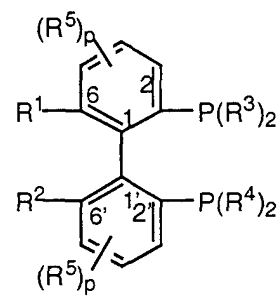

V

oder der allgemeinen Formel

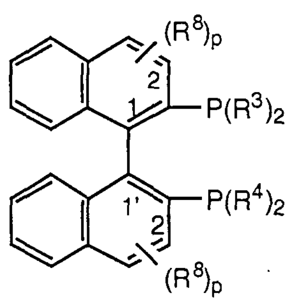

VI

worin

$R^1$ und $R^2$ unabhängig voneinander $C_1$-$C_4$- Alkyl, $C_1$-$C_4$- Alkoxy, Di-($C_1$-$C_4$-Alkyl)amino, Hydroxy, geschütztes Hydroxy, Hydroxymethyl oder geschütztes Hydroxymethyl,

oder

$R^1$ und $R^2$ zusammen eine zweiwertige Gruppe

$-(CH_2)_q-$ , $-CH_2-O-CH_2-$ , 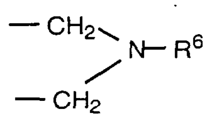

oder

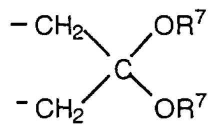

$R^3$ und $R^4$ unabhängig voneinander $C_1$-$C_4$- Alkyl, $C_{3-7}$-Cycloalkyl, Phenyl oder substituiertes Phenyl sind, wobei ein substituiertes Phenyl einen oder mehrere, gleiche oder verschiedene Substituenten ausgewählt aus $C_1$-$C_4$- Alkyl oder Halogen aufweist, oder $R^3$ und $R^4$ einen fünfgliedrigen Heteroaromaten oder eine Gruppe der Formel

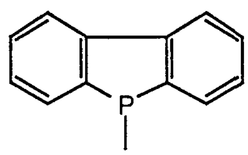

darstellen,

$R^5$ $C_1$-$C_4$- Alkyl oder $C_1$-$C_4$- Alkoxy,

$R^6$ $C_1$-$C_4$- Alkyl, Phenyl, Benzyl, substituiertes Phenyl oder substituiertes Benzyl ist, wobei eine substituierte Phenylgruppe einen oder mehrere, gleiche oder verschiedene Substituenten ausgewählt aus $C_1$-$C_4$-Alkyl oder Halogen aufweist und die substituierte Benzylgrupe am Phenylteil einen oder mehrere, gleiche oder verschiedene Substituenten ausgewählt aus Fluor, $C_1$-$C_4$-Alkyl, $C_1$-$C_4$-Alkoxy, Di-($C_1$-$C_4$-Alkyl)amino, Tri($C_1$-$C_4$-Alkyl)silyl und Phenyl aufweist,

$R^7$ $C_1$-$C_4$- Alkyl oder beide $R^7$ zusammen Di- oder Trimethylen,

$R^8$ Halogen, Hydroxy, Methyl, Aethyl, Amino, Acetamido, Nitro oder Sulfo,

p Null oder die Zahl 1, 2 oder 3

und q die Zahl 3, 4 oder 5 bedeuten.

Im Rahmen der obigen Definition der Formeln I-VI und in den nachfolgenden Erläuterungen bedeutet der Ausdruck "Halogenid" bzw. "Halogen" Fluor, Chlor, Brom oder Jod. Der Ausdruck "$C_1$-$C_4$-Alkyl" bedeutet geradkettige oder verzweigte Alkylgruppen mit 1 bis 4 Kohlenstoffatomen, wie Methyl, Aethyl, Propyl, Isopropyl, n-Butyl, Isobutyl und tert.Butyl. Der Ausdruck "$C_1$-$C_4$-Alkoxy" bedeutet Gruppen, in denen der Alkylrest die vorhergehende Bedeutung hat. Dies gilt auch für andere "$C_1$-$C_4$-Alkyl" enthaltende Gruppen, wie halogeniertes $C_1$-$C_4$-Alkyl und Di($C_1$-$C_4$-Alkyl)-amino. Unter der soeben erwähnten Gruppe "halogeniertes $C_1$-$C_4$-Alkyl" sind $C_1$-$C_4$-Alkylgruppen zu verstehen, die einfach oder mehrfach mit gleichen oder verschiedenen Halogenatomen substituiert sind, und zwar insbesondere mit Fluor und/oder Chlor. Vorzugsweise befindet sich ein Halogenatom in $\alpha$-Stellung (am Rest A) zur $-COO^{\ominus}$. Bevorzugte halogenierte $C_1$-$C_4$- Alkylgruppen sind perchlorierte und perfluorierte $C_1$-$C_4$- Alkylgruppen, z.B. Trichlormethyl bzw. Pentafluoräthyl. Falls der Rest $R^3$, $R^4$ oder $R^6$ gegebenenfalls substituiertes Phenyl oder, bezüglich $R^6$ allein, gegebenenfalls substituiertes Benzyl bedeutet, kommen hier in Frage als Substituenten (im Falle von Benzyl, für dessen Phenylteil) insbesondere Fluor, $C_1$-$C_4$-Alkyl- oder Alkoxygruppen (vorzugsweise Methyl bzw. Methoxy), Di($C_1$-$C_4$-Alkyl) amino (vorzugsweise Dimethylamino), Tri($C_1$-$C_4$-Alkyl)silyl (vorzugsweise Trimethylsilyl) und Phenyl. Als Schutzgruppen für die Hydroxy-bzw. Hydroxymethylgruppe ($R^1$ und/oder $R^2$ als geschütztes Hydroxy oder geschütztes Hydroxymethyl) kommen insbesondere in Betracht die üblichen Aether-bildenden Gruppen, wie z.B. Benzyl, Allyl, Benzyloxymethyl, niederes Alkoxymethyl oder auch 2-Methoxyäthoxymethyl und dergleichen. Der Ausdruck "fünfgliedriger Heteroaromat" steht für einen Substituenten der Formel

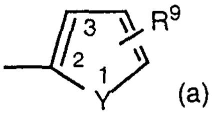 (a) 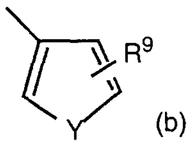 (b) 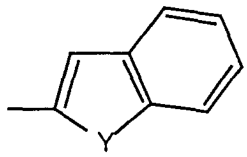 (c) 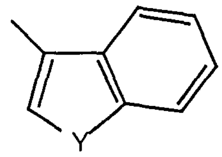 (d)

worin Y Sauerstoff, Schwefel oder $NR^{10}$; $R^9$ Wasserstoff, $C_1$-$C_4$-Alkyl (insbesondere Methyl) oder $C_1$-$C_4$ - Alkoxy (insbesondere Methoxy); und $R^{10}$ $C_1$-$C_4$-Alkyl (insbesondere Methyl) bedeuten.

Falls p in der Formel VI eine Zahl 1 bis 3 bedeutet, befinden sich die beiden bzw. zumindest zwei Reste $R^8$ vorzugsweise in 5,5'-Stellung.

Die erfindungsgemässe asymmetrische Hydrierung der Verbindung der Formel II oder eines Salzes davon zur Verbindung der Formel I kann in einem geeigneten, unter den Reaktionsbedingungen inerten organischen Lösungsmittel erfolgen. Als derartige Lösungsmittel kommen insbesondere in Betracht niedere Alkohole, wie beispielsweise Methanol und Aethanol; aliphatische Ester, wie beispielsweise Aethylacetat; halogenierte aliphatische Kohlenwasserstoffe, wie beispielsweise Methylenchlorid; cyclische Aether, wie beispielsweise Tetrahydrofuran und Dioxan; und Wasser; und auch Gemische obiger Lösungsmittel. Die Hydrierung wird zweckmässigerweise bei Temperaturen zwischen etwa 0°C und etwa 150°C, vorzugsweise im Temperaturbereich von etwa 20°C bis etwa 100°C, und bei einem Druck von etwa 1 bis etwa 100 bar, vorzugsweise von etwa 5 bis etwa 40 bar, durchgeführt. Das prozentuale Molverhältnis von Ruthenium im Ruthenium-Katalysator zu der zu hydrierenden Verbindung der Formel II bzw. zu deren Salz (dem "Substrat") liegt zweckmässigerweise zwischen etwa 0,0005 und etwa 5 [entspricht einem Molverhältnis Substrat:Katalysator (S/C) von etwa 200.000 bis etwa 20], vorzugsweise zwischen etwa 0,001 und etwa 0,01 (S/C etwa 100.000 bis etwa 10.000).

Falls als Ausgangsmaterial ein Salz der Verbindung der Formel II verwendet wird, kann dieses als solches eingesetzt oder in situ, beispielsweise durch Zusatz von ca. einem Moläquivalent (bezogen auf die Menge Edukt) einer Base zum Hydrierungsmedium, erzeugt werden. Als Basen eignen sich insbesondere Alkali- und Erdalkalimetallhydroxide, z.B. Natriumhydroxid, Kaliumhydroxid bzw. Calciumhydroxid; tert.Ammoniumhydroxide, z.B. Triäthylammoniumhydroxid; quaternäre Ammoniumhydroxide, z.B. Tetramethylammoniumhydroxid und Tetrabutylammoniumhydroxid; sowie Alkylamine, z.B. Triäthylamin.

Die Aufarbeitung erfolgt auch im Falle eines Salzes in der Regel extrem einfach, beispielsweise durch Kristallisation aus der mit einer Mineralsäure, z.B. Salzsäure, angesäuerten, teilweise eingeengten Hydrierlösung. Mittels Umkristallisation kann das Produkt in guter Reinheit erhalten werden.

Die optisch aktiven Ruthenium-Diphosphin-Komplexe, z.B. diejenigen der Formeln III und IV, sind entweder bekannt oder können in an sich bekannter Weise hergestellt werden, beispielsweise in Analogie zu den in der Europäischen Patentpublikation 397.042 und in J. Chem. Soc. Chem. Commun. 1989, 1208-1210 (K. Mashima et al.) beschriebenen Verfahren. Die Liganden der Formeln V und VI selbst sind bekannt, z.B. aus den europäischen Patentpublikationen 104.375 und 398.132 bzw. aus der japanischen Patentpublikation (Kokai) 136.605/1978, oder können in Analogie zu der Herstellung der bekannten Liganden erhalten werden. Diese Publikationen beinhalten Methoden zur Herstellung derjenigen Liganden der Formeln V und VI, in denen $R^3$ und $R^4$ gleich sind. Diejenigen Verbindungen, worin $R^3$ und $R^4$ voneinander verschieden sind, können analog hierzu erhalten werden, allerdings in zwei Stufen, beispielsweise gemäss folgendem Reaktionsschema:

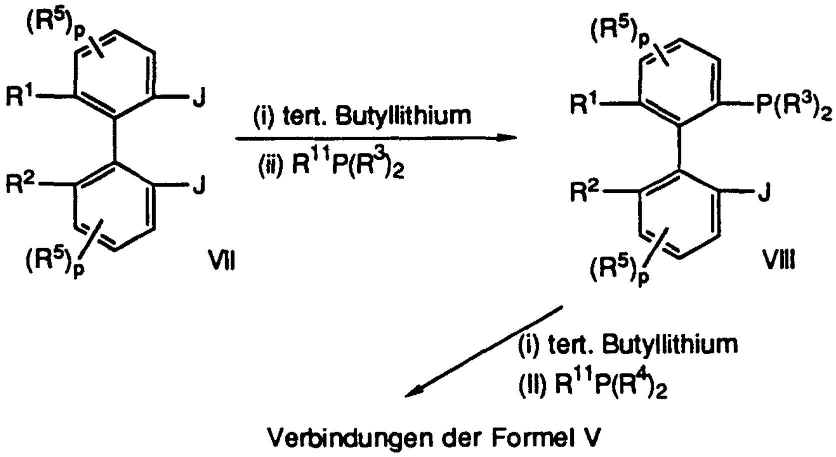


, worin $R^1$, $R^2$, $R^3$, $R^4$, $R^5$ und p die oben angegebenen Bedeutungen besitzen und $R^{11}$ eine Abgangsgruppe bedeutet, z.B. Halogen, insbesondere Chlor oder Brom; oder Alkoxy, insbesondere Methoxy oder Aethoxy. Um zu gewährleisten, dass jeweils nur ein Jodatom durch ein Lithiumatom ersetzt wird, werden die entsprechenden Reaktionen zweckmässigerweise mit etwa äquivalenten Mengen an Reaktionspartnern durchgeführt.

Bei der Durchführung der erfindungsgemässen asymmetrischen Hydrierung kann der optisch aktive Ruthenium-Diphosphin-Komplex, z.B. derjenige der Formel III oder IV, zuerst hergestellt werden, und dann eine Lösung der zu hydrierenden Verbindung der Formel II oder ein Salz davon zugegeben werden. Alternativ kann der Ruthenium-Katalysator jedoch in situ hergestellt werden, und zwar wunschgemäss in Gegenwart oder in Abwesenheit der zu hydrierenden Verbindung.

Die in dem erfindungsgemässen Verfahren als Ausgangsmaterialien verwendeten 3-Carboxy-3-pyrazolin-, 3-Carboxy-1,2,5,6-tetrahydropyridazin-bzw. 3-Carboxy-1,2-diaza-3-cyclohepten-Derivate der allgemeinen Formel II und deren Salze sind neu und bilden einen weiteren Gegenstand der vorliegenden Erfindung. Diese Verbindungen können beispielsweise gemäss folgendem Reaktionsschema hergestellt werden:

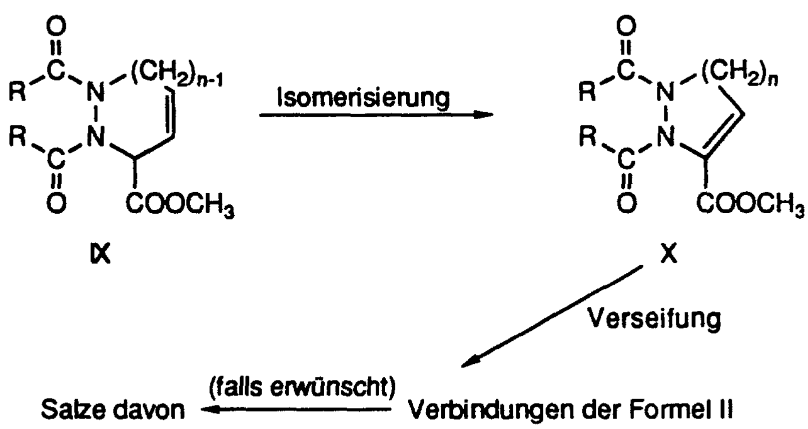


worin R und n die oben angegebenen Bedeutungen besitzen. Die Verbindungen der Formel IX sind entweder bekannt oder können nach an sich bekannten Methoden hergestellt werden.

Die Isomerisierung der Verbindung der Formel IX zur Verbindung der Formel X erfolgt zweckmässigerweise in einem organischen Lösungsmittel bei Temperaturen von etwa Raumtemperatur bis zur Rückflusstemperatur des Reaktionsgemisches, und zudem in Gegenwart einer Base. Als organisches Lösungsmittel eignet sich insbesondere ein cyclischer Aether, z.B. Dioxan oder Tetrahydrofuran, oder ein aromatischer Kohlenwasserstoff, z.B. Toluol, und als geeignete Base insbesondere 1,5-Diazabicyclo[4.3.0]non-5-en, 1,8-Diazabicyclo[5.4.0]undec-7-en oder Kaliumtert. butylat.

Die nachfolgende Verseifung wird zweckmässigerweise unter Einwirkung von Essigsäure oder Ameisensäure durchgeführt. Das Reaktionsgemisch wird geeigneterweise in Gegenwart eines Katalysators, wie beispielsweise

Trifluormethansulfonsäure, Salzsäure, Bromwasserstoffsäure, Schwefelsäure, Methansulfonsäure, Trifluorborätherat oder Trifluormethansulfonsäure-trimethylsilylester, und bei Temperaturen zwischen etwa 60°C und der Rückflusstemperatur des Reaktionsgemisches durchgeführt. Man verwendet zweckmässigerweise eine 4-120%-molare Menge des Katalysators bezogen auf die Menge Edukt (Verbindung der Formel X).

Falls nicht unmittelbar in Form eines Salzes hergestellt, kann die so erhaltene Verbindung der Formel II auf an sich bekannte Weise in das gewünschte Salz übergeführt werden. Ebenfalls auf an sich bekannte Weise kann ein so erhaltenes Salz der Verbindung der Formel II in die jeweilige Verbindung der Formel II übergeführt werden.

Als Alternative zur Isomerisierung der Verbindungen der Formel IX können die Verbindungen der Formel X gemäss folgendem Reaktionsschema hergestellt werden:

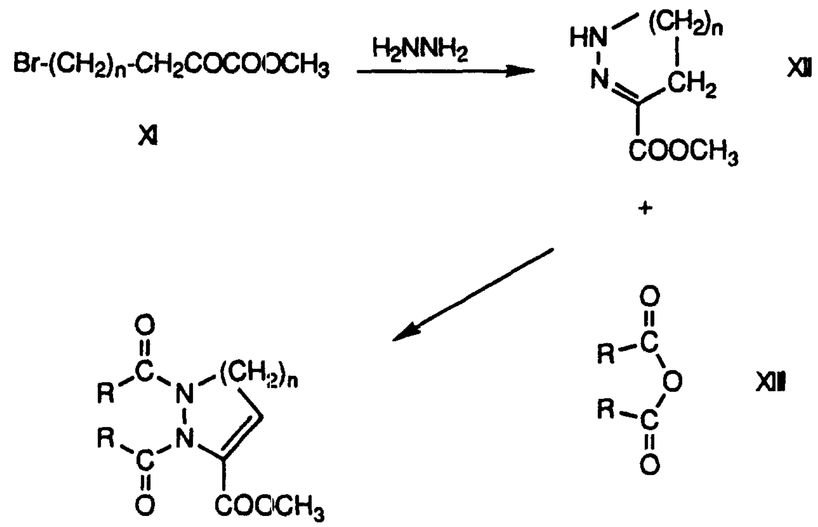


XI XII XIII

X

worin R und n die oben angegebenen Bedeutungen besitzen. Die Verbindungen der Formel XI sind entweder bekannt oder können nach an sich bekannten Methoden hergestellt werden.

Bei dem in der ersten Stufe (XI→XII) des alternativen Verfahrens verwendeten Hydrazin handelt es sich insbesondere um Hydrazinhydrat, wobei auch ein Salz von Hydrazin, z.B. das Hydrochlorid oder das Hydrosulfat, eingesetzt werden kann. Die diesbezügliche Umsetzung erfolgt zweckmässigerweise in einem alkoholischen Lösungsmittel bei Temperaturen von etwa 0°C bis zur Rückflusstemperatur des Reaktionsgemisches, und zudem gegebenenfalls in Gegenwart einer Base. Als alkoholisches Lösungsmittel eignet sich insbesondere Methanol, Aethanol, Isopropanol oder ein wässriges Gemisch eines solchen Alkohols, z.B. ein Gemisch von Methanol und Wasser, das aus bis zu 90 Gewichtsprozent, vorzugsweise aus von 10 bis 25 Gewichtsprozent Wasser, besteht. Man verwendet als Base insbesondere ein niederes Trialkylamin, z.B. Triäthylamin; einen Stickstoffenthaltenden Heterozyklus, z.B. Pyridin; ein Alkalimetallcarbonat oder -hydrogencarbonat, z.B. Kaliumcarbonat bzw. Natriumhydrogencarbonat; oder ein Alkalimetallhydroxid, z.B. Natriumhydroxid. Zu diesem Zweck erweist sich Kaliumcarbonat als besonders geeignet. Als günstige Bedingungen für die in der Reaktion involvierte Hydrazonbildung erweisen sich eine Reaktionstemperatur von ca. 5°C und ein pH-Bereich von 4 bis 7. Eingestellt wird dieser pH-Bereich zweckmässigerweise durch anfängliche Zugabe von Salzsäure oder vorzugsweise Essigsäure zur Hydrazinhydrat-Lösung und während der Reaktion durch langsame Zugabe von Kaliumcarbonat-Lösung. Um die anschliessende Ueberführung des intermediär gebildeten Hydrazons in die cyclische Verbindung der Formel XII zu fordern, wird das Reaktionsgemisch zweckmässigerweise bei dem gleichen pH-Wert auf Rückflusstemperatur erhitzt, wobei hier die langsame Zugabe einer weiteren Menge Base erforderlich sein kann.

Bei der nachfolgenden Umsetzung der Verbindung der Formel XII mit dem Säureanhydrid der Formel XIII, z. B. Phthalsäureanhydrid (die beiden R bilden 1,2-Phenylene) handelt es sich um eine Kondensation (Abspaltung von Wasser). Diese Umsetzung erfolgt zweckmässigerweise unter Einwirkung von Thionylchlorid, mit oder ohne Base, oder von Essigsäureanhydrid in Gegenwart einer Base, in einem organischen Lösungsmittel und bei Temperaturen zwischen etwa der Raumtemperatur und der Rückflusstemperatur des Reaktionsgemisches. Als Base eignet sich insbesondere ein niederes Trialkylamin, z.B. Triäthylamin, oder ein Stickstoff-enthaltender Heterozyklus, z.B. Pyridin. Man verwendet als Lösungsmittel geeigneterweise einen niederen chlorierten Kohlenwasserstoff, z.B. Methylenchlorid, einen niederen aliphatischen Ester, z.B. Aethylacetat, oder einen aromatischen Kohlenwasserstoff, z.B. Toluol. Vorzugsweise verwendet man Methylenchlorid.

Bei allen oben beschriebenen Verfahrensstufen erfolgt die Aufarbeitung und Isolierung des jeweiligen Produktes auf an sich bekannte Weise.

Das Verfahren zur Herstellung der Ausgangsmaterialien der Formel II (beide mehrstufige Verfahren) stellt einen weiteren Gegenstand der vorliegenden Erfindung dar.

Das erfindungsgemässe Hydrierverfahren erlaubt die Herstellung der Verbindungen der Formel I in hoher optischer Reinheit. Es wird besonders bevorzugt für die Herstellung von (S)-1,2,3,4,6,11-Hexahydro-6,11-dioxopyridazino[1,2-b]phthalazin-1-carbonsäure (Verbindung der Formel I, in der die beiden R zusammen 1,2-Phenylen und n 2 bedeuten, also der Formel

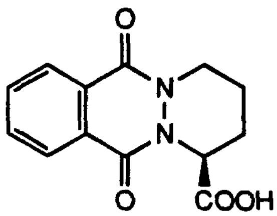

(S-Form)

) eingesetzt. Diese Verbindung ist ein wertvolles Zwischenprodukt in der Synthese des unter dem Namen "Cilazapril" bekannten blutdrucksenkenden Mittels.

Die folgenden Beispiele dienen zur Erläuterung der Erfindung. In diesen Beispielen haben die verwendeten Abkürzungen folgende Bedeutung:

| | |
|---|---|
| OAc | Acetyloxy |
| TFA | Trifluoracetyloxy |
| COD | (Z,Z)-1,5-Cyclooctadien |
| GC | Kapillar-Gaschromatographie; die Proben werden mit Bistrimethylsilylacetamid silyliert und auf einer PVMS-54 Permaphase-Säule (Perkin-Elmer) chromatographiert. |
| e.e. | Enantiomeric excess (enantiomerer Ueberschuss). Der e.e. der Hydrierprodukte wird durch Hochdruck-Flüssigkeitschromatographie (HPLC) auf einer α-APG-Säule ("α-acid glycoprotein, chiral-AGP" der Firma Chromtek, Schweden) bestimmt. |
| o.p. | Optische Reinheit |
| S/C | Substrat/Katalysator-Molverhältnis |
| Dehydrophthaloylsäure | 3,4,6,11-Tetrahydro-6,11-dioxo-pyridazino[1,2-b]-phthalazin-1-carbonsäure |
| (S)-Säure (R)-Säure | (S)- bzw. (R)-1,2,3,4,6,11-Hexahydro-6,11-dioxopyridazino[1,2-b]phthalazin-1-carbonsäure |
| BIPHEMP | (6,6'-Dimethylbiphenyl-2,2'-diyl)bis(diphenylphosphin) |
| MeOBIPHEP | (6,6'-Dimethoxybiphenyl-2,2'-diyl)bis(diphenylphosphin) |
| (3,4,5-MeO)-MeOBIPHEP | (6,6'-Dimethoxybiphenyl-2,2'-diyl)bis[di-(3,4,5-trimethoxyphenyl)phosphin] |
| ThienylBIPHEMP | (6,6'-Dimethylbiphenyl-2,2'-diyl)bis[di-(2-thienyl)-phosphin] |
| TriMeOBIPHEP | (4,4',5,5',6,6'-Hexamethoxybiphenyl-2,2'-diyl)bis-(diphenylphosphin) |
| DiMeOBIPHEP | (5,5',6,6'-Tetramethoxybiphenyl-2,2'-diyl)bis(diphenylphosphin) |
| BIPHOMP | (5,7-Dihydro-dibenz[c,e]oxepin-1,11-diyl)bis(diphenylphosphin) |
| p-TolMeOBIPHEP | (6,6'-Dimethoxybiphenyl-2,2'-diyl)bis[di-(p-tolyl)-phosphin] |
| p-TolBIPHEMP | (6,6'-Dimethylbiphenyl-2,2'-diyl)bis[di-(p-tolyl)phosphin] |
| pTolBINAP | (1,1'-Binaphthyl-2,2'-diyl)bis[di-(p-tolyl)phosphin] |
| $Cy_4$MeOBIPHEP | (6,6'-Dimethoxybiphenyl-2,2'-diyl)bis(dicyclohexylphosphin) |

Ferner sind alle Temperaturen in Grad Celsius (°C) angegeben.

Beispiel 1

a) In einer Glove-Box (Sauerstoffgehalt < 1 ppm) werden in einem 50 ml Messkolben 16,5 mg (0,0192 mMol)

Ru(OAc)$_2$[(S)-p-TolMeOBIPHEP] in 50 ml Methanol bei 20° gelöst, und die Lösung wird 10 Minuten gerührt, wobei sich eine klare, orange Lösung (die Katalysatorlösung) bildet.

b) In einer Glove-Box (Sauerstoffgehalt < 1 ppm) wird ein 500 ml Autoklav mit 8,0 g (31,0 mMol) Dehydrophthaloylsäure, 3,14 g (31,0 mMol) Triäthylamin, 148 ml Methanol und 2 ml der obenerwähnten Katalysatorlösung beladen. Die Hydrierung wird bei 60°, einem konstanten Druck von 40 bar reinem Wasserstoff und unter intensivem Rühren durchgeführt. Nach einer Stunde beträgt der Umsatz gemäss GC 100%. Die gelbe Hydrierlösung wird am Rotationsverdampfer bei 50°/220 mbar bis auf ein Gewicht von 25 g eingedampft. Zu der verbleibenden Lösung werden unter Rühren bei 20-35° 4,42 ml 25%-ige Salzsäure-Lösung und anschliessend 27 ml Wasser zugetropft. Die (S)-Säure beginnt bei ca. 25° auszufallen. Die Suspension wird 1 Stunde bei 20° und 1 Stunde bei 0° gerührt. Nach Filtration und Trocknen erhält man 7,7 g (96%) (S)-Säure als fast weisse Kristalle, $[\alpha]_{436}$ = -833,0° (c=1, Methanol); optische Reinheit 98,9%; 100% e.e.

Beispiele 2-11

Analog dem in Beispiel 1 beschriebenen Verfahren werden Hydrierungen jeweils unter Verwendung eines Ruthenium-Katalysators der Formel III $[Ru(X^1)_2L]$ durchgeführt, und zwar zwecks Umwandlung der Dehydrophthaloylsäure in die (S)-Säure. Der jeweilige Katalysator, das S/C, die Hydrierzeit und die optische Reinheit (o.p.) bzw. der enantiomere Ueberschuss (e.e.) sind in der nachfolgenden Tabelle angegeben.

Tabelle

| Beispiel | Verwendeter Katalysator $Ru(X^1)_2L$ | | S/C | Hydrierzeit bis Erreichen eines 100%-igen Umsatzes, in Stunden | o.p.% bzw. e.e.% |
|---|---|---|---|---|---|
| | $X^1$ | L | | | |
| 2 | OAc | (S)-ThienylBIPHEMP | 30.000 | 20 | 97,8(o.p.) |
| 3 | OAc | (S)-BIPHOMP | 30.000 | 5 | 98,6(o.p.) |
| 4 | OAc | (S)-TriMeOBIPHEP | 30.000 | 5 | 97,3(o.p.) |
| 5 | OAc | (S)-DiMeOBIPHEP | 30.000 | 5 | 98,6(o.p.) |
| 6 | OAc | (S)-MeOBIPHEP | 40.000 | 5 | 98,9(o.p.) |
| 7 | OAc | (S)-(3,4,5-MeO) MeOBIPHEP | 20.000 | 5 | 98,5(o.p.) |
| 8 | OAc | (S)-pTolBINAP | 40.000 | 20 | 98,7(o.p.) |
| 9 | OAc | (S)-pTolBIPHEMP | 40.000 | 5 | 98,5(o.p.) |
| 10 | $CH_3SO_2O$ | (S)-BIPHEMP | 400 | 1 | 98,6(o.p.) |
| 11 | TFA | (S)-pTolMeOBIPHEP | 50.000 | 20 | 96,4(e.e.) |

Die obigen o.p.-Werte sind diejenigen der kristallisierten (S)-Säure, während für Beispiel 11 der e.e.-Wert derjenige des Rohproduktes ist. Bei den Beispielen 2-10 betragen die Ausbeuten an kristallisierter Säure zwischen 90% und 95%.

Beispiel 12

In einer Glove-Box (Sauerstoffgehalt < 1 ppm) werden in einem 50 ml Schlenkrohr 25,35 mg (0,0775 mMol) Ru(COD)(OAc)$_2$ und 47,0 mg (0,0775 mMol) (S)-Cy$_4$MeOBIPHEP in 6 ml Diäthyläther und 2 ml Tetrahydrofuran gelöst, und die Lösung wird 1,5 Stunden bei 40° gerührt. Es bildet sich eine klare, rote Katalysatorlösung, welche in die Hydrierung eingesetzt wird.

Die Hydrierung und Aufarbeitung erfolgt wie in Beispiel 1b) beschrieben.

Man erhält 7,4 g (92%) (S)-Säure als fast weisse Kristalle, 99,2% e.e.

Beispiel 13

In einer Glove-Box (Sauerstoffgehalt < 1 ppm) werden 68,8 mg (0,0775 mMol) [Ru((S)-MeOBIPHEP)(p-Cymol)Cl]Cl in 50 ml Methanol bei 20° gelöst, und die Lösung wird 10 Minuten gerührt. Es bildet sich eine klare, orangerote Katalysatorlösung, welche in die Hydrierung eingesetzt wird.

Die Hydrierung und Aufarbeitung erfolgt wie in Beispiel 1b) beschrieben.

Man erhält 7,3 g (91%) (S)-Säure als fast weisse Kristalle, 100% e.e.

Beispiel 14

In einer Glove-Box (Sauerstoffgehalt < 1 ppm) werden 77,49 mg (0,0775 mMol) [Ru((S)BIPHEMP)(p-Cymol) $CH_3CN][BF_4]_2$ in 50 ml Methanol bei 20° gelöst, und die Lösung wird 10 Minuten gerührt. Es bildet sich eine klare, orangerote Katalysatorlösung, welche in die Hydrierung eingesetzt wird.
Die Hydrierung und Aufarbeitung erfolgt wie in Beispiel 1b) beschrieben.
Man erhält 6,8 g (85%) (S)-Säure als fast weisse Kristalle, 98,8% e.e.

Beispiel 15

a) In einer Glove-Box (Sauerstoffgehalt < 1 ppm) werden 10,63 mg (0,0124 mMol) $Ru(OAc)_2$[(S)-p-TolMeO-BIPHEP] in 50 ml Methanol bei 20° gelöst, und die Lösung wird 10 Minuten gerührt, wobei sich eine klare, orange Katalysatorlösung bildet.
b) Ein 500 ml Autoklav wird mit 32,0 g (123,9 mMol) Dehydrophthaloylsäure, 12,54 g (123,9 mMol) Triäthylamin, der obenerwähnten Katalysatorlösung, weiteren 13 ml Methanol und 7 ml Wasser beladen. Die Hydrierung wird bei 60°, einem konstanten Druck von 7 bar reinem Wasserstoff und unter intensivem Rühren durchgeführt. Nach 20 Stunden beträgt der Umsatz gemäss GC 100%. Zur Freisetzung der (S)-Säure wird ein aliquoter Teil der Hydrierlösung, enthaltend 0,5 g (S)-Säure, mit Methanol verdünnt und durch eine Säule mit Amberlyst® 15 fliessen gelassen. Die Säule wird mit Methanol nachgewaschen, bis alle Säure eluiert ist. Der e.e.-Wert der rohen Säure (0,5 g) beträgt 99,5%.

Beispiel 16

a) In einer Glove-Box (Sauerstoffgehalt < 1 ppm) werden in einem 50 ml Messkolben 13,3 mg (0,0155 mMol) $Ru(OAc)_2$[(S)-pTolMeOBIPHEP] in 50 ml Methanol bei 20° gelöst, und die Lösung wird 10 Minuten gerührt, wobei sich eine klare, orange Katalysatorlösung bildet.
b) Anschliessend wird in der Glove-Box ein 500 ml Autoklav mit 8,0 g (31,0 mMol) Dehydrophthaloylsäure, 140 ml Methanol und 10 ml der obenerwähnten Katalysatorlösung beladen. Die Hydrierung wird bei 60°, einem konstanten Druck von 7 bar reinem Wasserstoff und unter intensivem Rühren durchgeführt. Nach 23 Stunden beträgt der Umsatz gemäss GC 98%. Die gelbe, leicht trübe Hydrierlösung wird über Celite® (Kieselgur) filtriert, am Rotationsverdampfer bei 50°/220 mbar eingedampft und die hellgelbe, kristalline (S)-Säure bei 60°/0,01 mbar getrocknet. Die Ausbeute an (S)-Säure beträgt 7,7 g (96%), deren e.e.-Wert 99,1%.

Beispiel 17

In einer Glove-Box (Sauerstoffgehalt <1 ppm) wird ein 500 ml Autoklav mit 64,0 g (247,8 mMol) Dehydrophthaloylsäure, 25,08 g (247,8 mMol) Triäthylamin, 87,5 ml Methanol und 4,97 mg (0,0062 mMol) $Ru(OAc)_2$[(R)-MeOBIPHEP] beladen. Die Hydrierung erfolgt bei 100°C und einem konstanten Druck von 40 bar reinem Wasserstoff. Nach 6 Stunden Hydrierzeit beträgt der Umsatz gemäss GC 100%. Man dampft dann die Hydrierlösung teilweise ein und fällt als Hydrierprodukt die (R)-Säure durch Zutropfen von 34,4 ml 25%iger Salzsäure und 216 ml Wasser aus. Auf diese Weise erhält man 61,1 g (94,7% der Theorie) (R)-Säure als weisse Kristalle, 100% ee.

Beispiel 18

a) In einer Glove-Box (Sauerstoffgehalt <1 ppm) werden in einem 50 ml Schlenkrohr 10,14 mg (0,031 mMol) Di($\eta^2$-acetato)-($\eta^4$-cycloocta-1,5-dien)-ruthenium(II) [B. Heiser et al., Tetrahedron: Asymmetry 2, 51 (1991)] und 13,83 mg (0,031 mMol) [(S)-6,6'-Dimethoxybiphenyl-2,2'-diyl]bis(diisopropyl-phosphin) in 6 ml Diäthyläther und 2 ml Tetrahydrofuran gelöst und 1,5 Stunden bei 40° gerührt. Es bildet sich eine rote klare Katalysatorlösung.
Das als Ausgangsmaterial verwendete [(S)-6,6'-Dimethoxybiphenyl-2,2'-diyl]bis(diisopropylphosphin) wurde hergestellt durch eine Grignard-Reaktion zwischen (S)-(6,6'-Dimethoxybiphenyl-2,2'-diyl)bis(phosphonsäure-diphenylester) und 4-Isopropylmagnesiumbromid in Tetrahydrofuran, gefolgt von der Reduktion des daraus resultierenden (S)-(6,6'-Dimethoxy-biphenyl-2,2'-diyl)bis(diisopropylphosphinoxid) mit Trichlorsilan in Xylol in Gegenwart von Tributylamin.
b) Die Hydrierung erfolgt in einem 500 ml-Autoklaven, der mit 8,0 g (31,0 mMol) Dehydrophthaloylsäure, 3,14 g (31,0 mMol) Triäthylamin, 150 ml Methanol und mit der oben erwähnten Katalysatorlösung beladen wird. Die Hydrierung wird bei 60°, einem konstanten Druck von 40 bar reinem Wasserstoff und unter intensivem Rühren durchgeführt. Nach 5 Stunden beträgt der Umsatz gemäss GC 99,9%. Die gelbe Hydrierlösung wird am Rotationsverdampfer

bei 50°/220 mbar bis auf ein Gewicht von 25 g eingedampft. Zu der verbleibenden Lösung werden unter Rühren bei 20-35°4,42 ml 25-%ige Salzsäure-Lösung und. anschliessend 27 ml Wasser zugetropft. Die (S)-Säure beginnt bei ca. 25° auszufallen. Die Suspension wird 1 Stunde bei 20° und 1 Stunde bei 0° gerührt. Nach Filtration und Trocknung erhält man 7,7 g (96%) (S)-Säure als fast weisse Kristalle mit einer enantiomeren Reinheit von 96,5% e.e. Der e.e.-Wert wird durch HPLC auf einer α-APG-Säule bestimmt.

Beispiel 19

Herstellung des Zwischenproduktes 3.4.6.11-Tetrahydro-6.11-dioxo-pyridazino[1,2-b]phthalazin-1-carbonsäure-methylester (1. Variante)

Unter Argonbegasung und Rühren werden nacheinander 46,4 g (165,3 mMol; Reinheit ca. 97%) 1,4,6,11-Tetrahydro-6,11-dioxo-pyridazino[1,2-b]-phthalazin-1-carbonsäure-methylester und 1,27 ml (8,27 mMol; 5 Mol%; Reinheit ca. 97%) 1,8-Diazabicyclo[5.4.0]undec-7-en zu 450 ml Dioxan gegeben. Nach Erhöhung der Innentemperatur auf 50° wird die resultierende hellgelbe Lösung unter gleichbleibenden Bedingungen 16 Stunden gerührt, dann auf Raumtempertatur abgekühlt. Mittels Dünnschichtchromatographie und GC-Analyse erfolgt die Ueberprüfung der Einstellung des Reaktionsgleichgewichts: gemäss GC beträgt das Verhältnis 3,4,6,11-Verbindung (Produkt): 1,4,6,11-Verbindung (Ausgangsmaterial) 90,3:9,7.

Anschliessend wird die Innentemperatur auf 5° gesenkt. Ohne Rühren wird das Reaktionsgemisch 16 Stunden bei 5° stehen gelassen, dann erneut mittels Dünnschichtchromatographie und GC untersucht, mit dem Ergebnis, dass eine 100%ige Umwandlung 1,4,6,11- → 3,4,6,11-Verbindung festgestellt wird.

Nun wird der Festkörper durch Erwärmen auf 18° geschmolzen und die daraus entstandene Suspension sofort unter Rühren mit 6,28 g (33,0 mMol) p-Toluolsulfonsäurehydrat versetzt. Man rührt die hellbeige Suspension 3 Stunden unter langsamem Erwärmen auf Raumtempetatur nach und gibt anschliessend 2,72 g (33,2 mMol) wasserfreies Natriumacetat zu. Nach einstündigem Rühren wird das Reaktionsgemisch am Hochvakuum bei einer Badtemperatur von 25° eingedampft. Der feuchte Rückstand wird dann in 300 ml Methylenchlorid gelöst und die Lösung mit 10%iger wässriger Natriumchloridlösung gewaschen. Anschliessend trocknet man die vereinigten organischen Phasen über wasserfreiem Natriumsulfat und dampft das Filtrat mitsamt Methylenchlorid-Spülungen des abgefilterten Natriumsulfats unter vermindertem Druck (Wasserstrahlvakuum) bei ca. 40° bis zur Gewichtskonstanz ein. Auf diese Weise erhält man 45,8 g eines gelbbraunen Kristallisats, das gemäss GC aus nahezu 100%igem reinem 3,4,6,11-Tetrahydro-6,11-dioxo-pyridazino[1,2-b]phthalazin-1-carbonsäure-methylester besteht. Die Rohausbeute beträgt nahezu 100%.

Das Rohprodukt (45,8 g) wird in 250 ml heissem Methylenchlorid gelöst und das Gemisch ca. 65 Stunden bei 5° stehen gelassen. Nach dieser Periode wird die resultierende Suspension filtriert und der Rückstand auf der Nutsche mit 10 ml n-Hexan gewaschen und dann ca. 16 Stunden bei 50° am Wasserstrahlvakuum getrocknet. Man erhält auf diese Weise 40,3 g gelbe Kristalle, Smp. 160-161°, die gemäss GC aus 100%igem gewünschtem Produkt bestehen. Die Ausbeute beträgt 90%.

Nach Eindampfen der Mutterlauge zur Trockene erhält man weitere 6,6 g 3,4,6,11-Tetrahydro-6,11-dioxo-pyridazino[1,2-b]phthalazin-1-carbonsäure-methylester (nahezu 100%ig rein) als gelbbraune Kristalle.

Beispiel 20

Herstellung des Zwischenproduktes 3.4.6.11-Tetrahydro-6.11-dioxo-pyridazino[1,2-b]phthalazin-1-carbonsäure-methylester (2, Variante)

a) 363 ml (374 g, 7,47 Mol) Hydrazinhydrat werden unter Rühren in einem Gemisch aus 4,4l Methanol und 500 ml deionisiertem Wasser gelöst. Zu der Lösung werden nun bei Raumtemperatur innert 15 Minuten und unter fortgesetztem Rühren 120 ml (2,10 Mol) Eisessig zugetropft. Man kühlt die Lösung auf 4-6° ab und gibt bei dieser Temperatur innert 3 Stunden eine Lösung von 1587 g (6,60 Mol; ca. 87%ige Reinheit gemäss GC) rohem 5-Brom-2-oxo-pentansäure-methylester in 900 ml Methanol zu, wobei nach Zugabe von ca. Dreiviertel dieser Lösung 200 ml 3M wässrige Kaliumcarbonatlösung so zudosiert werden, dass der pH-Wert des Reaktionsgemisches zwischen 4 und 7 beträgt. Nach beendeter Zugabe der Lösung von 5-Brom-2-oxo-pentansäure-methylester wird unter fortgesetztem Rühren die Innentemperatur innert 30 Minuten auf 62-63° (Rückfluss) erhöht und der pH-Wert durch gleichzeitige Zugabe von 1,1l 3M Lösung von Kaliumcarbonat in deionisiertem Wasser zwischen 4 und 7 gehalten. Das Reaktionsgemisch wird nach beendeter Zugabe der Kaliumcarbonatlösung weitere 2,5 Stunden bei der Rückflusstemperatur gerührt.

Zur Aufarbeitung wird das Reaktionsgemisch am Wasserstrahlvakuum bei 40° eingeengt, wobei insgesamt ca. 5 l Methanol abdestilliert werden. Man verdünnt die zurückgebliebene braune Suspension mit 1l Wasser, wobei der salzartige Niederschlag aufgelöst wird, und extrahiert die wässrige Lösung fünfmal mit je 3l Aethylacetat. Jede

organische Phase wird nun mit jeweils 1l Wasser gewaschen, und die vereinigten organischen Phasen (ca. 5l) werden dann am Wasserstrahlvakuum bei 45° bis zur Gewichtskonstanz eingedampft. Es resultieren 1093 g einer gelben, kristallinen Masse. Dieses Rohprodukt wird in einem Gemisch aus 1 l Aethylacetat und 2 l Toluol gelöst und die resultierende Lösung zweimal mit jeweils 250 ml halbgesättigter wässriger Natriumchloridlösung gewaschen, durch Hyflo® (Filterhilfsmittel) filtriert und dann eingedampft. (Durch das Waschen mit Wasser werden polare, nicht näher identifizierte Nebenprodukte entfernt, die andernfalls die Reinheit des Endproduktes beeinträchtigt hätten).

Anschliessend versetzt man den Rückstand mit 500 ml eines 1:1-Gemisches aus Toluol und n-Hexan, rührt die resultierende Suspension kurz bei 50° und kühlt sie auf Raumtemperatur ab. Man nutscht dann die Kristalle ab, wässcht den Nutschkuchen mit einem Gemisch aus 250 ml Toluol und 250 ml n-Hexan und trocknet diesen am Wasserstrahlvakuum bei 50° bis zur Gewichtskonstanz. Daraus resultieren 610,7 g schwach gelbliche Kristalle des gewünschten Zwischenproduktes 1,4,5,6-Tetrahydro-3-pyridazincarbonsäure-methylester. Das Filtrat wird seinerseits eingedampft und der Rückstand mit 200 ml eines 1:1-Gemisches aus Toluol und n-Hexan versetzt, kurz bei Raumtemperatur gerührt und filtriert. Man wäscht den Nutschkuchen mit einem Gemisch aus 10 ml Toluol und 60 ml n-Hexan und trocknet ihn am Wasserstrahlvakuum bei 50°, woraus 45,3 g gelbe Kristalle weiteren Zwischenproduktes resultieren.

Das Filtrat wird zur Trockene eingedampft und der rotschwarze, ölige Rückstand (373 g) anschliessend am Molekularverdampfer bei 128°/0,04-0,06 mbar destilliert. Man löst das teilweise kristalline, gelbe Destillat (176 g) dann in 750 ml Aethylacetat, wäscht die Lösung mit 100 ml halbgesättigter, wässriger Natriumchloridlösung und dampft sie zur Trockene ein. Der Rückstand wird mit 150 ml eines 1:1-Gemisches aus Toluol und n-Hexan versetzt, kurz gerührt und dann filtriert, und der Nutschkuchen mit einem Gemisch aus 25 ml Toluol und 25 ml n-Hexan gewaschen und am Wasserstrahlvakuum bei 50° getrocknet. Es resultieren weitere 42,0 g gelbe Kristalle von 1,4,5,6-Tetrahydro-3-pyridazincarbonsäure-methylester.

Die gesamte Ausbeute an 1,4,5,6-Tetrahydro-3-pyridazincarbonsäure-methylester als gelblichen Kristallen beträgt 698 g (74% der theoretischen Ausbeute; gemäss Dünnschichtchromatographie und GC rein).

b) Ein Gemisch von 213,2 g (1,50 Mol) 1,4,5,6-Tetrahydro-3-pyridazin-carbonsäure-methylester [gemäss dem in a) beschriebenen Verfahren hergestellt], 240 g (1,62 Mol) Phthalsäureanhydrid und 21 Methylenchlorid wird unter Rühren bei 40° (Rückflusstemperatur) 17 Stunden erhitzt. Man kühlt die resultierende Suspension ab, versetzt sie bei 17-21° innert 110 Minuten mit einer Lösung von 140 ml (1,29 Mol) Thionylchlorid in 100 ml Methylenchlorid und überlässt sie anschliessend 2 Stunden der Nachreaktion.

Zur Aufarbeitung wird das Reaktionsgemisch am Wasserstrahlvakuum bei 40° weitgehend eingeengt und der feste Rückstand mit 1l Methanol versetzt, wobei eine gelbe, gut rührbare Suspension entsteht. Diese wird nun eingedampft und der Rückstand zweimal mit jeweils 500 ml Methanol versetzt und jedesmal eingedampft. Der resultierende gelbe Kristallbrei wird dann in 150 ml Methanol suspendiert und die Suspension filtriert. Zum Schluss wird der Nutschkuchen zweimal mit jeweils 300 ml Methanol aufgeschlämmt, jedesmal gut trockengesogen und am Wasserstrahlvakuum ca. 16 Stunden bei 50° getrocknet. Auf diese Weise erhält man 368,3 g (90% der theoretischen Ausbeute) 3,4,6,11-Tetrahydro-6,11-dioxo-pyridazino[1,2-b]phthalazin-1-carbonsäure-methylester als gelbliches, homogenes Kristallpulver, Smp. 156-157°. Das Produkt erweist sich gemäss Dünnschichtchromatographie als rein.

Beispiel 21

Herstellung von 3.4.6.11-Tetrahydro-6,11-dioxo-pyridazino[1,2-b]phthalazin-1-carbonsäure

Unter Argonbegasung und Rühren werden bei 50° 75 g (275 mMol) 3,4,6,11-Tetrahydro-6,11-dioxo-pyridazino [1,2-b]phthalazin-1-carbonsäure-methylester (gemäss dem in Beispiel 19 oder 20 beschriebenen Verfahren hergestellt) in 300 ml Essigsäure gelöst. Dann versetzt man die Lösung mit 5 g Darco® KB-B (Aktivkohle in Pulverform; ICI), erhöht die Innentemperatur auf ca. 65° und rührt die resultierende Suspension bei dieser Temperatur 30 Minuten. Anschliessend wird die warme Suspension durch ca. 10 g Dicalite® (Filterhilfsmittel; Dicalite Europe Nord S.A.) filtriert und das Dicalite® -Polster mit 150 ml Essigsäure gewaschen. Die vereinigten Essigsäurephasen werden am Wasserstrahlvakuum bei 50-55° zum Teil eingedampft, und die zurückgebliebene gelbe Lösung wird mit 80 ml Essigsäure verdünnt. Nun versetzt man diese Lösung mit 1 ml (11,4 mMol) Trifluormethansulfonsäure und rührt die Lösung 20 Stunden bei 102°. (Die anfänglich klare, gelbe Lösung trübt sich nach ca. 30 Minuten, wonach sich ein heller Niederschlag, der allmählich dicker wird, bildet, und zwar innert wenigen Minuten. Die Suspension bleibt durchwegs gut rührbar).

Zur Aufarbeitung wird das Reaktionsgemisch auf 25° abgekühlt, mit 400 ml deionisiertem Wasser versetzt und 1,5 Stunden bei Raumtemperatur gerührt. Man filtriert nun die Suspension und saugt den Nutschkuchen gut trokken. Zum Schluss wird der Nutschkuchen auf der Nutsche dreimal mit jeweils 180 ml deionisiertem Wasser, dann zweimal mit jeweils 250 ml Methanol, gut gewaschen, jedesmal gut trockengesogen und im Trockenschrank am Wasserstrahlvakuum bei 55° ca. 16 Stunden getrocknet. Auf diese Weise erhält man 62,6 g (88% der theoretischen Ausbeute) 3,4,6,11-Tetrahydro-6,11-dioxo-pyridazino[1,2-b]phthalazin-1-carbonsäure, die gemäss GC eine Reinheit von 98 Flächenprozent aufweist.

## Patentansprüche

1. Verfahren zur Herstellung von Verbindungen der allgemeinen Formel

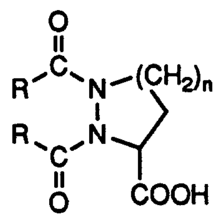

I

worin
jedes

R unabhängig Alkyl, Arylmethyl, Aryl, Alkoxy, Arylmethoxy oder Aryloxy ist oder beide R zusammen Methylen, Aethylen oder 1,2-Phenylen bedeuten, wobei der Term Aryl allein oder in Kombination Phenyl oder substituiertes Phenyl ist, wobei substituiertes Phenyl ein Phenyl mit einem oder mehreren, gleichen oder verschiedenen Substituenten ausgewählt aus $C_1$-$C_6$-Alkyl, $C_1$-$C_6$-Alkoxy oder Halogen ist; und
n 1, 2 oder 3 bedeutet,

in der (S)- oder (R)-Form, dadurch gekennzeichnet, dass man das entsprechende 3-Carboxy-3-pyrazolin-, 3-Carboxy-1,2,5,6-tetrahydropyridazin- bzw. 3-Carboxy-1,2-diaza-3-cyclohepten-Derivat der allgemeinen Formel

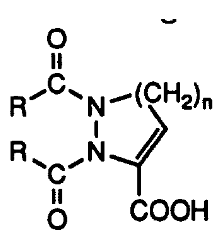

II

worin die beiden R und n die oben angegebenen Bedeutungen besitzen, oder ein Salz davon
in Gegenwart eines optisch aktiven Ruthenium-Diphosphin-Komplexes asymmetrisch hydriert,
wobei die Umsetzung von 3,4,6,11-Tetrahydro-6,11-dioxo-pyridazo[1,2a]-phthalazin-1-carbonsäure zu (S)-1,2,3,4,6,11-Hexahydro-6,11-dioxo-pyridazo[1,2b]phthalazin-1-carbonsäure mittels eines Katalysator erhältlich aus Di($\eta^2$-acetato-($\eta^4$-cycloocta-1,5-dien)ruthenium (II) und [(S)-6,6'-Dimethoxybiphenyl-2,2'-diyl]bis[diisopropylphosphin] ausgeschlossen ist.

2. Verfahren nach Anspruch 1, dadurch gekennzeichnet, dass man als optisch aktiven Ruthenium-Diphosphin-Komplex einen Komplex der allgemeinen Formel

$$Ru(X^1)_2L \qquad II$$

oder

$$[Ru(X^2)_{2-m}X^3L](X^4)_m \qquad IV$$

verwendet, worin

$X^1$ Halogenid, ein Anion A-$COO^{\ominus}$ oder ein Anion A-$SO_2O^{\ominus}$,
$X^2$ Halogenid,
$X^3$ Benzol, Hexamethylbenzol oder p-Cymol,
$X^4$ Halogenid, $BF_4{}^{\ominus}$, $ClO_4{}^{\ominus}$ oder B(Phenyl)$_4{}^{\ominus}$,
m die Zahl 1 oder 2 ist,
A $C_1$-$C_4$-Alkyl, halogeniertes $C_1$-$C_4$-Alkyl oder Aryl ist, wobei der Term Aryl ein Phenyl, Biphenyl, Naphthyl, substituiertes Phenyl, substituiertes Biphenyl oder substituiertes Naphthyl ist, wobei die substituierten Gruppen einen oder mehrere, gleiche oder verschiedene Substituenten ausgewählt aus $C_1$-$C_4$-Alkyl oder Halogen sind und
L einen optisch aktiven atropisomeren Diphosphinliganden bedeutet.

**3.** Verfahren nach Anspruch 2, dadurch gekennzeichnet, dass der optisch aktive atropisomere Diphosphinligand L einer der allgemeinen Formel

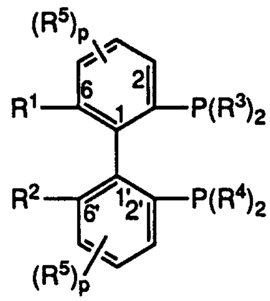

V

oder der allgemeinen Formel

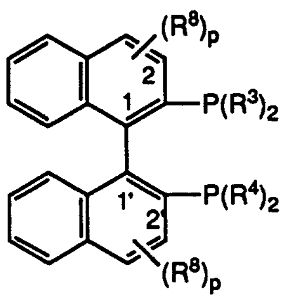

VI

ist, worin

$R^1$ und $R^2$ unabhängig voneinander $C_1$-$C_4$-Alkyl, $C_1$-$C_4$-Alkoxy, Di-($C_1$-$C_4$-Alkyl)amino, Hydroxy, geschütztes Hydroxy, Hydroxymethyl oder geschütztes Hydroxymethyl,

oder

$R^1$ und $R^2$ zusammen eine zweiwertige Gruppe

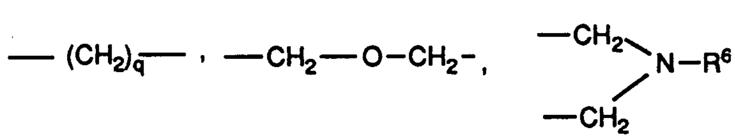

oder

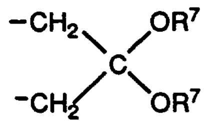

$R^3$ und $R^4$ unabhängig voneinander $C_1$-$C_4$-Alkyl, $C_{3-7}$-Cycloalkyl, Phenyl oder substituiertes Phenyl sind, wobei ein substituiertes Phenyl einen oder mehrere, gleiche oder verschiedene Substituenten ausgewählt aus $C_1$-$C_4$-Alkyl oder Halogen aufweist, oder $R^3$ und $R^4$ einen fünfgliedrigen Heteroaromaten oder eine Gruppe der Formel

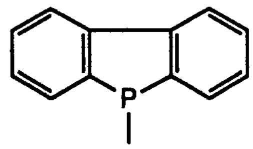

darstellen,

$R^5$ $C_1$-$C_4$-Alkyl oder $C_1$-$C_4$-Alkoxy,

$R^6$ $C_1$-$C_4$-Alkyl, Phenyl, Benzyl, substituiertes Phenyl oder substituiertes Benzyl ist, wobei eine substituierte Phenyl-Gruppe einen oder mehrere, gleiche oder verschiedene Substituenten ausgewählt aus $C_1$-$C_4$-Alkyl oder Halogen aufweist und die substituierte Benzyl-Gruppe am Phenylteil einen oder mehrere, gleiche oder verschiedene Substituenten ausgewählt aus Fluor, $C_1$-$C_4$-Alkyl-, $C_1$-$C_4$-Alkoxy, Di($C_1$-$C_4$-Alkyl)amino, Tri($C_1$-$C_4$-Alkyl)silyl und Phenyl aufweist und

$R^7$ $C_1$-$C_4$-Alkyl oder beide $R^7$ zusammen Di- oder Trimethylen,

$R^8$ Halogen, Hydroxy, Methyl, Aethyl, Amino, Acetamido, Nitro oder Sulfo,

p Null oder die Zahl 1, 2 oder 3

und q die Zahl 3, 4 oder 5 bedeuten.

**4.** Verfahren nach einem der Ansprüche 1 bis 3, dadurch gekennzeichnet, dass man 3,4,6,11-Tetrahydro-6,11-dioxo-pyridazino[1,2-b]phthalazin-1-carbonsäure zur (S)-1,2,3,4,6,11-Hexahydro-6,11-dioxo-pyridazino-[1,2-b]phthalazin-1-carbonsäure hydriert.

**5.** Verbindungen der allgemeinen Formel

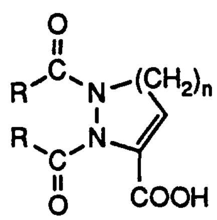

II

worin
jedes

R unabhängig Alkyl, Arylmethyl, Aryl, Alkoxy, Arylmethoxy oder Aryloxy ist oder beide R zusammen Methylen, Aethylen oder 1,2-Phenylen bedeuten, wobei der Term Aryl allein oder in Kombination Phenyl oder substituiertes Phenyl ist, wobei substituiertes Phenyl ein Phenyl mit einem oder mehreren, gleichen oder verschiedenen Substituenten ausgewählt aus $C_1$-$C_6$-Alkyl, $C_1$-$C_6$-Alkoxy oder Halogen ist; und

n 1, 2 oder 3 bedeuten,

wobei 3,4,6,11-Tetrahydro-6,11-dioxo-pyridazo [1,2a]-phthalazin-1-carbonsäure ausgeschlossen ist, und deren Salze.

**6.** Verfahren zur Herstellung von Verbindungen der allgemeinen Formel

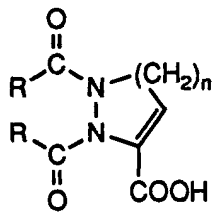

II

worin
jedes

R unabhängig Alkyl, Arylmethyl, Aryl, Alkoxy, Arylmethoxy oder Aryloxy oder beide R zusammen Methylen, Aethylen oder 1,2-Phenylen bedeuten, wobei der Term Aryl allein oder in Kombination Phenyl oder substituiertes Phenyl ist, wobei substituiertes Phenyl ein Phenyl mit einem oder mehreren, gleichen oder verschiedenen Substituenten ausgewählt aus $C_1$-$C_6$-Alkyl, $C_1$-$C_6$-Alkoxy oder Halogen ist; und

n 1,2 oder 3 bedeuten,

und von deren Salzen, dadurch gekennzeichnet, dass man eine Verbindung der allgemeinen Formel

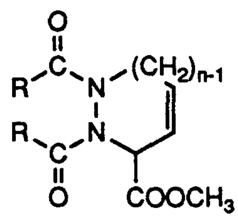

IX

worin R und n die oben angegebenen Bedeutungen besitzen, zu der entsprechenden Verbindung der allgemeinen Formel

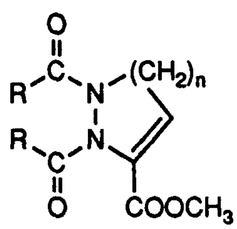

X

isomerisiert, und diese Verbindung zur Verbindung der allgemeinen Formel II verseift, und gegebenenfalls letztere Verbindung auf an sich bekannte Weise in das gewünschte Salz überführt.

**7.** Verfahren zur Herstellung von Verbindungen der allgemeinen Formel

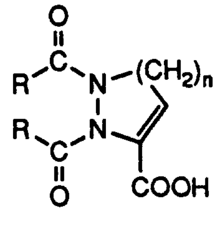

II

worin
jedes

R unabhängig Alkyl, Arylmethyl, Aryl, Alkoxy, Arylmethoxy oder Aryloxy oder beide R zusammen Methylen, Aethylen oder 1,2-Phenylen bedeuten, wobei der Term Aryl allein oder in Kombination Phenyl oder substituiertes Phenyl ist, wobei substituiertes Phenyl ein Phenyl mit einem oder mehreren, gleichen oder verschiedenen Substituenten ausgewählt aus $C_1$-$C_6$-Alkyl, $C_1$-$C_6$-Alkoxy oder Halogen ist; und
n 1,2 oder 3 bedeuten,

und von deren Salzen, dadurch gekennzeichnet, dass man einen Brom-2-oxo-alkansäure-methylester der allgemeinen Formel

$$Br\text{-}(CH_2)_n\text{-}CH_2COCOOCH_3 \qquad XI$$

worin n 1,2 oder 3 bedeutet,
mit Hydrazin umsetzt, die resultierende Verbindung der allgemeinen Formel

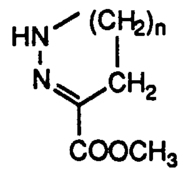

XII

worin n die oben angegebene Bedeutung besitzt,
mit einem Säureanhydrid der allgemeinen Formel

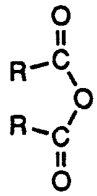

XIII

worin R die oben angegebene Bedeutung besitzt,
zur Verbindung der allgemeinen Formel

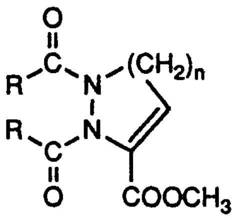

X

umsetzt und diese Verbindung zur Verbindung der allgemeinen Formel II verseift, und gegebenenfalls letztere Verbindung auf an sich bekannte Weise in das gewünschte Salz überführt.

## Claims

**1.** A process for the manufacture of compounds of the general formula

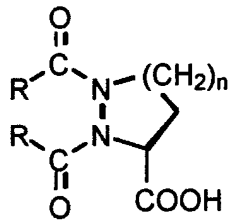

I

wherein
each

R independently signifies alkyl, arylmethyl, aryl, alkoxy, arylmethoxy or aryloxy or both R's together signify methylene, ethylene or 1,2-phenylene, whereby the term aryl alone or in combination is phenyl or substituted phenyl, whereby substituted phenyl is a phenyl with one or more, similar or different substituents selected from $C_1$-$C_6$-alkyl, $C_1$-$C_6$-alkoxy or halogen; and
n signifies 1,2 or 3,

in the (S)- or (R)-form, characterized by asymmetrically hydrogenating the corresponding 3-carboxy-3-pyrazoline, 3-carboxy-1,2,5,6-tetrahydropyridazine or 3-carboxy-1,2-diaza-3-cycloheptene derivative of the general formula

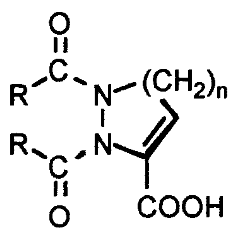

II

wherein the two R's and n have the significances given above,
or a salt thereof
in the presence of an optically active ruthenium-diphosphine complex,
whereby the conversion of 3,4,6,11-tetrahydro-6,11-dioxo-pyridazo[1,2a]phthalazine-1-carboxylic acid into (S)-1,2,3,4,6,11-hexahydro-6,11-dioxo-pyridazo[1,2b]phthalazine-1-carboxylic acid using a catalyst obtainable from di($\eta^2$-acetate-($\eta^4$-cycloocta-1,5-diene)-ruthenium (II) and [(S)-6,6'-dimethoxybiphenyl-2,2'-diyl]bis[diisopropylphosphine) is excluded.

**2.** A process according to claim 1, characterized in that a complex of the general formula

$$Ru(X^1)_2L \qquad III$$

or

$$[Ru(X^2)_{2-m}X^3L](X^4)_m \qquad IV$$

wherein

$X^1$ signifies halide, an anion A-$COO^{\ominus}$ or an anion A-$SO_2O^{\ominus}$,
$X^2$ signifies halide,
$X^3$ signifies benzene, hexamethylbenzene or p-cymene,
$X^4$ signifies halide, $BF_4^{\ominus}$, $ClO_4^{\ominus}$ or B(phenyl)$_4^{\ominus}$,

m signifies the number 1 or 2,

A signifies $C_1$-$C_4$-alkyl, halogenated $C_1$-$C_4$-alkyl or aryl, whereby the term aryl is a phenyl, biphenyl, naphthyl, substituted phenyl, substituted biphenyl or substituted naphthyl, whereby the substituted groups are one or more, similar or different substituents selected from $C_1$-$C_4$-alkyl or halogen, and

L signifies an optically active atropisomeric diphosphine ligand is used as the optically active ruthenium-diphosphine complex.

**3.** A process according to claim 2, characterized in that the optically active atropisomeric diphosphine ligand L is one of the general formula

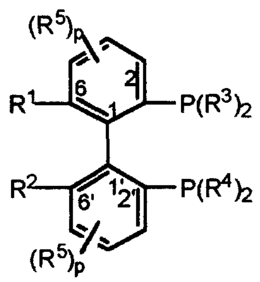

V

or of the general formula

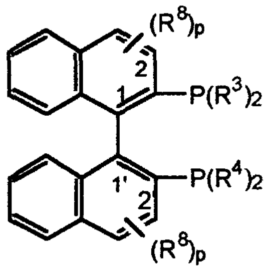

VI

wherein

$R^1$ and $R^2$ each independently signify $C_1$-$C_4$-alkyl, $C_1$-$C_4$-alkoxy, di($C_1$-$C_4$-alkyl)-amino, hydroxy, protected hydroxy, hydroxymethyl or protected hydroxymethyl

or

$R^1$ and $R^2$ together signify a divalent group

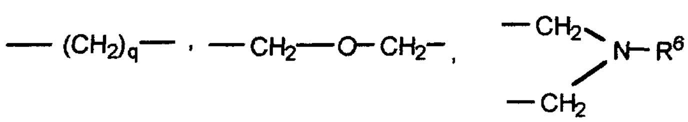

or

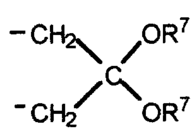

$R^3$ and $R^4$ are each independently $C_1$-$C_4$-alkyl, $C_{3-7}$-cycloalkyl, phenyl or substituted phenyl, whereby a substituted phenyl has one or more, similar or different substituents selected from $C_1$-$C_4$-alkyl or halogen, or $R^3$ and $R^4$ represent a five-membered heteroaromatic or a group of the formula

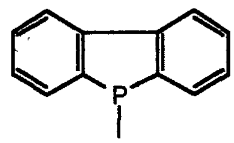

| | |
|---|---|
| $R^5$ | signifies $C_1$-$C_4$-alkyl or $C_1$-$C_4$-alkoxy, |
| $R^6$ | signifies $C_1$-$C_4$-alkyl, phenyl, benzyl, substituted phenyl or substituted benzyl, whereby a substituted phenyl group has one or more, similar or different substituents selected from $C_1$-$C_4$- alkyl or halogen and the substituted benzyl group has on the phenyl part one or more, similar or different substituents selected from fluorine, $C_1$-$C_4$-alkyl, $C_1$-$C_4$-alkoxy, di($C_1$-$C_4$-alkyl)amino, tri($C_1$-$C_4$-allcyl)silyl and phenyl and |
| $R^7$ | signifies $C_1$-$C_4$-alkyl or both $R^7$'s together signify di- or trimethylene, |
| $R^8$ | signifies halogen, hydroxy, methyl, ethyl, amino, acetamido, nitro or sulpho, |
| p | signifies zero or the number 1,2 or 3 |
| and q | signifies the number 3, 4 or 5. |

**4.** A process according to any one of claims 1 to 3, characterized in that 3,4,6,11-tetrahydro-6,11-dioxo-pyridazino [1,2-b]phthalazine-1-carboxylic add is hydrogenated to (S)-1,2,3,4,6,11 -hexahydro-6,11-dioxo-pyridazino[1,2-b] phthalazine-1-carboxylic acid.

**5.** Compounds of the general formula

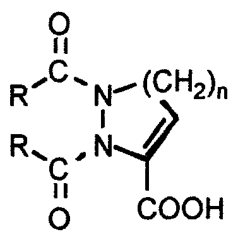

II

wherein
each

R independently signifies alkyl, arylmethyl, aryl, alkoxy, arylmethoxy or aryloxy or both R's together signify methylene, ethylene or 1,2-phenylene, whereby the term aryl alone or in combination is phenyl or substituted phenyl, whereby substituted phenyl is a phenyl with one or more, similar or different substituents selected from $C_1$-$C_6$-alkyl, $C_1$-$C_6$-alkoxy or halogen; and

n signifies 1,2 or 3,

whereby 3,4,6,11-tetrahydro-6,11-dioxo-pyridazo[1,2a] -phthalazine-1-carboxylic acid is excluded, and their salts.

**6.** A process for the production of compounds of the general formula

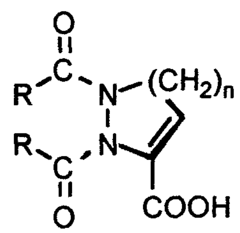

II

wherein
each

R independently signifies alkyl, arylmethyl, aryl, alkoxy, arylmethoxy or aryloxy or both R's together signify methylene, ethylene or 1,2-phenylene, whereby the term aryl alone or in combination is phenyl or substituted phenyl, whereby substituted phenyl is a phenyl with one or more, similar or different substituents selected from $C_1$-$C_6$-alkyl, $C_1$-$C_6$-alkoxy or halogen; and

n signifies 1,2 or 3,

and of their salts, characterized by isomerizing a compound of the general formula

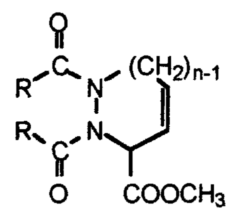

IX

wherein R and n have the significances given above, to the corresponding compound of the general formula

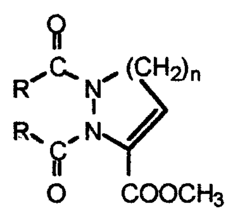

X

wherein R and n have the significances given above,
and saponifying this compound to the compound of general formula II and, if desired, converting the latter compound into the desired salt in a manner known per se.

**7.** A process for the production of compounds of the general formula

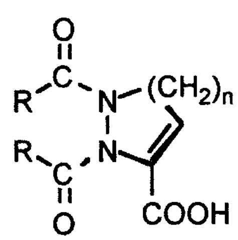

II

wherein
each

R independently signifies alkyl, arylmethyl, aryl, alkoxy, arylmethoxy or aryloxy or both R's together signify methylene, ethylene or 1,2-phenylene, whereby the term aryl alone or in combination is phenyl or substituted phenyl, whereby substituted phenyl is a phenyl with one or more, similar or different substituents selected from $C_1$-$C_6$-alkyl, $C_1$-$C_6$-alkoxy or halogen; and
n signifies 1,2 or 3,

and of their salts, characterized by reacting a methyl 2-oxo-bromoalkanoate of the general formula

$$Br\text{-}(CH_2)_n\text{-}CH_2COCOOCH_3 \qquad XI$$

wherein n signifies 1,2 or 3,
with hydrazine, converting the resulting compound of the general formula

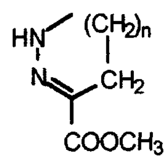

XI

wherein n has the significance given above,
with an acid anhydride of the general formula

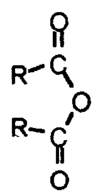

XII

wherein R has the significance given above,
into the compound of the general formula

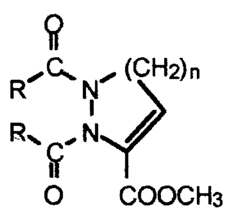

X

wherein R and n have the significances given above,
and saponifying this compound to the compound of general formula II and, if desired, converting the latter compound into the desired salt in a manner known per se.

## Revendications

**1.** Procédé pour la préparation de composés de formule générale

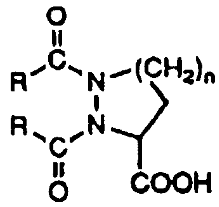

I

dans laquelle

R représentent chacun, indépendamment l'un de l'autre, un groupe alkyle, arylméthyle, aryle, alcoxy, arylméthoxy ou aryloxy, ou les deux radicaux R forment ensemble un groupe méthylène, éthylène ou 1,2-phénylène, le fragment aryle, seul ou en combinaison, étant le groupe phényle ou un groupe phényle substitué, le groupe phényle substitué étant un groupe phényle portant un ou plusieurs substituants identiques ou différents, choisis parmi des atomes d'halogène et des groupes alkyle en $C_1$-$C_6$ et alcoxy en $C_1$-$C_6$ ; et
n représente 1, 2 ou 3,

sous la forme (S) ou (R), caractérisé en ce que l'on soumet à une hydrogénation asymétrique le dérivé correspondant de 3-carboxy-3-pyrazoline, 3-carboxy-1,2,5,6-tétrahydropyridazine ou 3-carboxy-1,2-diaza-3-cyclo-heptène de formule générale

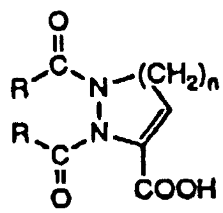

II

dans laquelle les deux radicaux R et n ont les significations données ci-dessus,
ou un sel de celui-ci,
en présence d'un complexe diphosphine-ruthénium,
à l'exclusion de la conversion de l'acide 3,4,6,11-tétrahydro-6,11-dioxo-pyddazo[1,2a]phtalazine-1-carboxylique en l'acide (S)-1,2,3,4,6,11-hexahydro-6,11-dioxo-pyridazo[1,2b]phtalazine-1-carboxylique, au moyen d'un catalyseur pouvant être obtenu à partir de di($\eta^2$-acétato-($\eta^4$-cyclo-octa-1,5-diène)ruthénium-(II) et [(S)-6,6'-diméthoxybiphényl-2,2'-diyl]-bis[diisopropylphosphine].

**2.** Procédé selon la revendication 1, caractérisé en ce que l'on utilise comme complexe ruthénium-diphosphine un complexe de formule générale

$$Ru(X^1)_2L \qquad III$$

ou

$$[Ru(X^2)_{2-m}X^3L](X^4)_m \qquad IV$$

formules dans lesquelles

$X^1$ représente un halogénure, un anion $A\text{-}COO^{\ominus}$ ou un anion $A\text{-}SO_2O^{\ominus}$,
$X^2$ représente un halogénure,
$X^3$ représente le benzène, l'hexaméthylbenzène ou le p-cymène,
$X^4$ représente un halogénure, $BF_4^{\ominus}$, $ClO_4^{\ominus}$ ou $B(phényle)_4^{\ominus}$,
m est le nombre 1 ou 2,

A représente un groupe alkyle en $C_1$-$C_4$, alkyle en $C_1$-$C_4$ halogéné ou aryle, le groupe aryle étant un groupe phényle, biphényle, naphtyle, phényle substitué, biphényle substitué ou naphtyle substitué, les substituants étant un ou plusieurs substituants identiques ou différents, choisis parmi des groupes alkyle en $C_1$-$C_4$ et des atomes d'halogène, et

L représente un ligand diphosphine atropisomère optiquement actif.

**3.** Procédé selon la revendication 2, caractérisé en ce que le ligand diphosphine L atropisomère optiquement actif est un ligand de formule générale

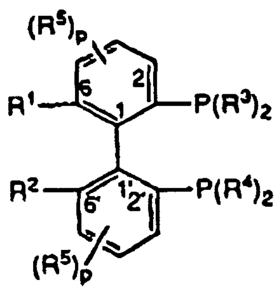

V

ou de formule générale

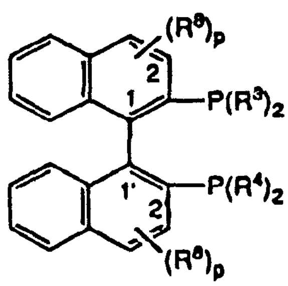

VI

formules dans lesquelles

$R^1$ et $R^2$ représentent, indépendamment l'un dc l'autre, un groupe alkyle en $C_1$-$C_4$, alcoxy en $C_1$-$C_4$, dialkyl ($C_1$-$C_4$)amino, hydroxy, hydroxy protégé, hydroxyméthyle ou hydroxyméthyle protégé,

ou

$R^1$ et $R^2$ forment ensemble un groupe divalent

$-(CH_2)_q-$ , $-CH_2-O-CH_2-$, 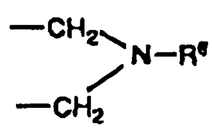

ou

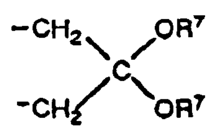

$R^3$ et $R^4$ représentent, indépendamment l'un de l'autre, un groupe alkyle en $C_1$-$C_4$, cycloalkyle en $C_3$-$C_7$, phényle ou phényle substitué, un groupe phényle substitué portant un ou plusieurs substituants identiques ou différents, choisis parmi des atomes d'halogène et des groupes alkyle en $C_1$-$C_4$, ou $R^3$ et $R^4$ forment

ensemble un cycle hétéroaromatique à 5 chaînons ou un groupe de formule

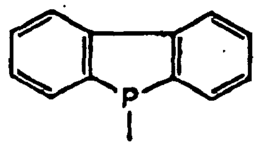

- $R^5$ représente un groupe alkyle en $C_1$-$C_4$ ou alcoxy en $C_1$-$C_4$,
- $R^6$ représente un groupe alkyle en $C_1$-$C_4$, phényle, benzyle, phényle substitué ou benzyle substitué, un groupe phényle substitué portant un ou plusieurs substituants identiques ou différents, choisis parmi des groupes alkyle en $C_1$-$C_4$ et des atomes d'halogène, et le groupe benzyle substitué sur le fragment phényle portant un ou plusieurs substituants identiques ou différents, choisis parmi l'atome de fluor et des groupes alkyle en $C_1$-$C_4$, alcoxy en $C_1$-$C_4$, dialkyl($C_1$-$C_4$)amino, trialkyl($C_1$-$C_4$)silyle et phényle, et
- $R^7$ représente un groupe alkyle en $C_1$-$C_4$, ou les deux symboles $R^7$ représentent ensemble le groupe di- ou triméthylène,
- $R^8$ représente un atome d'halogène ou le groupe hydroxy, méthyle, éthyle, amino, acétamido, nitro ou sulfo,
- p représente zéro ou le nombre 1, 2 ou 3, et
- q représente le nombre 3, 4 ou 5.

**4.** Procédé selon l'une des revendications 1 à 3, caractérisé en ce que l'on soumet l'acide 3,4,6,11-tétrahydro-6,11-dioxo-pyridazino[1,2-b]phtalazine-1-carboxylique à une hydrogénation conduisant à l'acide (S)-1,2,3,4,6,11-hexahydro-6,11-dioxo-pyridazino[1,2-b]phtalazine-1-carboxylique.

**5.** Composés de formule générale

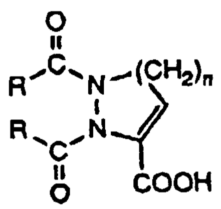

II

dans laquelle

- R représentent chacun, indépendamment l'un de l'autre, un groupe alkyle, arylméthyle, aryle, alcoxy, arylméthoxy ou aryloxy, ou les dcux radicaux R forment ensemble un groupe méthylène, éthylène ou 1,2-phénylène, le fragment aryle, seul ou en combinaison, étant le groupe phényle ou un groupe phényle substitué, le groupe phényle substitué étant un groupe phényle portant un ou plusieurs substituants identiques ou différents, choisis parmi des atomes d'halogène et des groupes alkyle en $C_1$-$C_6$ et alcoxy en $C_1$-$C_6$; et
- n représente 1, 2 ou 3,

à l'exclusion de l'acide 3,4,6,11-tétrahydro-6,11-dioxo-pyridazo[1,2a]-phtalazine-1-carboxylique, et leurs sels.

**6.** Procédé pour la préparation de composés de formule générale

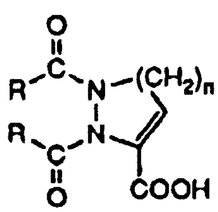

II

dans laquelle

R représentent chacun, indépendamment l'un de l'autre, un groupe alkyle, arylméthyle, aryle, alcoxy, arylméthoxy ou aryloxy, ou les deux radicaux R forment ensemble un groupe méthylène, éthylène ou 1,2-phénylène, le fragment arylc, seul ou en combinaison, étant le groupe phényle ou un groupe phényle substitué, le groupe phényle substitué étant un groupe phényle portant un ou plusieurs substituants identiques ou différents, choisis parmi des atomes d'halogène et des groupes alkyle en $C_1$-$C_6$ et alcoxy en $C_1$-$C_6$ ; et

n représente 1, 2 ou 3,

et de leurs sels,
caractérisé en ce que l'on soumet à une isomérisation un composé de formule générale

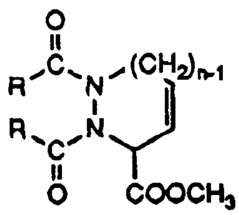 IX

dans laquelle R et n ont les significations indiquées,
pour aboutir au composé correspondant de formule générale

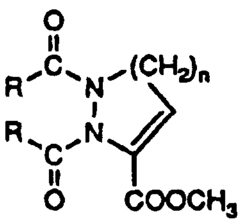 X

et on saponifie ce composé en le composé de formule générale II, et
éventuellement ce dernier composé est converti, d'une façon connue en soi, en le sel recherché.

**7.** Procédé pour la préparation de composés de formule générale

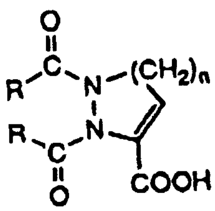 II

dans laquelle

R représentent chacun, indépendamment, un groupe alkyle, aryl-méthyle, aryle, alcoxy, arylméthoxy ou aryloxy, ou les deux radicaux R forment ensemble un groupe méthylène, éthylène ou 1,2-phénylène, le fragment aryle, seul ou en combinaison, étant le groupe phényle ou un groupe phényle substitué, le groupe phényle substitué étant un groupe phényle portant un ou plusieurs substituants identiques ou différents, choisis parmi des atomes d'halogène et des groupes alkyle en $C_1$-$C_6$ et alcoxy en $C_1$-$C_6$; et

n représente 1, 2 ou 3,

et de leurs sels, caractérisé en ce que l'on fait réagir avec de l'hydrazine un bromo-2-oxo-alcanoate de méthyle de formule générale

$$Br\text{-}(CH_2)_n\text{-}CH_2COCOOCH_3 \qquad XI,$$

on fait réagir le composé résultant, de formule générale

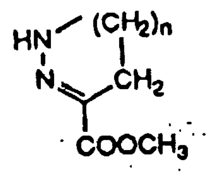

XII

dans laquelle n a la signification donnée plus haut,
avec un anhydride d'acide de formule générale

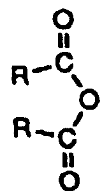

XIII

dans laquelle R a la signification donnée plus haut,
pour aboutir au composé de formule générale

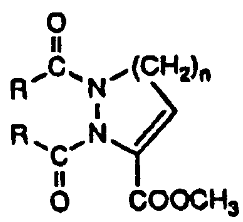

X

et on saponifie ce composé pour aboutir au composé de formule générale II, et éventuellement on convertit ce dernier composé, d'une façon connue en soi, en le sel recherché.